# EUROPEAN PATENT SPECIFICATION

(11) **EP 2 604 684 B1**
(45) Date of publication and mention of the grant of the patent: **10.08.2016**
(21) Application number: 12196315.1
(22) Date of filing: 10.12.2012
(51) Int. Cl.: C12M 1/12, A61L 27/56, C12N 5/00

(54) **Production method of tissue regeneration material and tissue regeneration material**
Herstellungsverfahren von Geweberegenerierungsmaterial und Geweberegenerierungsmaterial
Procédé de production de matériau de régénération tissulaire et matériau de régénération tissulaire

(30) Priority: 16.12.2011 JP 2011275555
(43) Date of publication of application: 19.06.2013
(73) Proprietor: GC Corporation, Bunkyo-ku Tokyo 113-0033 (JP)
(72) Inventor: Yamanaka, Katsuyuki, Tokyo, 174-8585 (JP); Yamamoto, Katsushi, Tokyo, 174-8585 (JP); Sakai, Yuuhiro, Tokyo, 174-8585 (JP); Shigemitsu, Yusuke, Tokyo, 174-8585 (JP)
(74) Representative: Beck Greener

(56) References cited:
- EP-A1- 1 541 182
- EP-A1- 2 080 800
- CHEN G ET AL: "Chondrogenic differentiation of human mesenchymal stem cells cultured in a cobweb-like biodegradable scaffold", BIOCHEMICAL AND BIOPHYSICAL RESEARCH COMMUNICATIONS, ACADEMIC PRESS INC. ORLANDO, FL, US, vol. 322, no. 1, 10 September 2004 (2004-09-10), pages 50-55, XP004526699, ISSN: 0006-291X, DOI: 10.1016/J.BBRC.2004.07.071
- SALGADO ANTONIO J ET AL: "Bone tissue engineering: State of the art and future trends", MACROMOLECULAR BIOSCIENCE, WILEY VCH VERLAG, WEINHEIM, DE, vol. 4, no. 8, 9 August 2004 (2004-08-09), pages 743-765, XP002485214, ISSN: 1616-5187, DOI: 10.1002/MABI.200400026

## Description

### Technical Field

The present invention relates to a production method of a tissue regeneration material in which to introduce a cell suspension into a porous support (porous body) and seed the cells thereon, and relates to a tissue regeneration material.

### Background Art

A tissue regeneration material formed by seeding cells onto a porous body, which is a supporting body having an interconnected porous structure, has been used in the field of tissue engineering. It is important that the porous body has small pores for culturing cells which communicate with one another three-dimensionally and extends deep into the central part thereof.

In reality, it is difficult to permeate cells, a culture medium, or a liquid mixed with cells and a culture medium (hereinafter sometimes referred to as a "cell suspension"), completely into the small pores of the porous body. Simply dripping the cell suspension onto the porous body or immersing the porous body into the cell suspension does not enable the cell suspension to reach the central part of the porous body.

In this regard, Patent Document 1 suggests, for example in claim 8, a method comprising sinking a porous body into a cell suspension, holding the porous body under reduced pressure or under vacuum, and thereby permeating the cell suspension into the porous body.

Patent Documents 2 and 3 disclose a method comprising immersing a porous body with water, replacing the water with a cell suspension simultaneously, and thereby introducing the cell suspension into the porous body.

Patent Document 4 discloses a method in which pores formed in a porous body are made relatively large to introduce a cell suspension into the pores easily, and a thickening agent such as collagen and gelatin are added to the cell suspension so that the cell suspension can be held in the porous body even with such relatively large pores. It is thought that this enables the cell suspension to be introduced and kept into the porous body.

### Citation List

### Patent Documents

Patent Document 1: Japanese Patent Application Laid-Open (JP-A) No. 2006-508717
Patent Document 2: JP-A No. 2007-181459
Patent Document 3: JP-A No. 2009-195682
Patent Document 4: JP-A No. 2003-038635

### Summary of the Invention

### Problems to be Solved by the Invention

Nevertheless, with the technique described in Patent Document 1, it is in fact difficult to permeate the cell suspension into the whole porous body even in the use of negative pressure. Therefore, a pathway for impregnation of the cell suspension needs to be formed by a laser beam or the like. Further, changing the pressure drastically by using negative pressure is not always good for the cells.

The techniques disclosed in Patent Documents 2 and 3 enable seeding of cells onto the whole porous body; however in these techniques, there is a drawback that some of the cell suspension flows out, resulting in an inaccurate number of cells introduced.

Especially in the techniques described in Patent Documents 1 to 3, the cell suspension mainly composed of water cannot permeate sufficiently into the central part of the porous body because of the relatively small pores of the porous body. In this respect, if a porous body having a pore structure of relatively large pores (with a pore diameter of 180 to 3500 µm and an average pore diameter of 350 to 2000 µm) is used, it is possible to easily permeate the cell suspension into the central part of the porous body. However, such a large pore diameter causes the cell suspension to easily pass through the porous body, making it difficult to retain the cell suspension inside the porous body.

On the other hand, according to the technique described in Patent Document 4, the cell suspension can easily permeate into the central part of the porous body. However, since the thickening agent remains inside the porous body, it is difficult to change a culture medium.

Cheng et al (Biochem. and Biophys. Res. Comm., 2004, 322, 50-55) discloses the use of a hybrid scaffold - a cobweb-like hybrid mesh of poly(DL-lactic-co-glycolic acid) and collagen - and the culture of human mesenchymal stem cells in said scaffold.

EP 2080800 discloses a porous cell scaffold having a thicknes of 2mm or more and having an internal small hole structure wholly seeded with cells, and a production method thereof.

EP 1541182 discloses a three dimensional porous scaffold suited for regeneration of living tissues, a process for producing said scaffold, and culturing cells or precursor cells in said scaffold.

Accordingly, an object of the present invention is to provide a production method of a tissue regeneration material by which a cell suspension can be introduced stably without being wasted, into a porous body having relatively large pores (with a pore diameter of 180 to 3500 µm and an average pore diameter of 350 to 2000 µm), that is, pores that make it difficult to keep the cell suspension inside the porous body, and the cells can be seeded on the porous body. Further, the present invention provides a tissue regeneration material.

### Means for Solving the Problems

The present invention will be described below. In order to make the present invention easy to understand, reference numerals given in the accompanying drawings are shown here in parentheses. However, the present invention is not limited to the embodiments shown in the drawings.

A first aspect of the present invention is a production method of a tissue regeneration material comprising: contacting a bottom surface of a porous body (11) to a surface of a holding plate (1) being a resin plate or a glass plate having a contact angle to water of 15 to 90°, the porous body being formed of a bioabsorbable polymer material selected from the group consisting of polyglycolic acid, polylactic acid and a copolymer of lactic acid and glycolic acid, poly-ε-caprolactone, a copolymer of lactic acid and ε-caprolactone, polyamino acid, polyortho ester, and a copolymer thereof, said porous body having a thickness of 2 to 100 mm, an area of the bottom surface of the porous body to be contacted with a holding plate of 0.5 to 200 cm², an interconnected porous structure with a pore diameter of 180 to 3500 µm and an average pore diameter of 350 to 2000 µm, a porosity of 60 to 95%, and a compressive strength of 0.05 to 1 MPa, and being filled with a cell suspension (12) having a cell density of 5 × 10⁶ to 1 × 10⁸ cell/ml; and the method further comprising reversing the holding plate and the porous body comprising the cell suspension such that the holding plate is on the upper side of the porous body when seen in the gravity direction, and keeping the porous body still in the air in a state that the weight of the holding plate is not applied to the porous body, to thereby seed cells on the porous body.

A second aspect of the present invention is the production method according to the first aspect, wherein the porosity of the porous body (11) is 80 to 90%.

A third aspect of the present invention is the production method according to the first or second aspect, wherein the average pore diameter of the porous body (11) is 540 to 1200 µm.

### Effects of the Invention

According to the present invention, it is possible to introduce a cell suspension into a porous body having relatively large pores, that is, pores that make it difficult to keep the cell suspension thereinside, and to seed the cells onto the porous body stably without wasting them.

### Brief Description of the Drawings

Fig. 1 is a view illustrating a process of a production method S10 of a tissue regeneration material: Fig. 1A shows a placement step; and Fig. 1B shows a cell suspension introduction step.
Fig. 2 is another view illustrating a process of the production method S10 of a tissue regeneration material: Fig. 2A shows a scene where a porous body 11 comprising a cell suspension 12 is placed on a holding plate 1; and Fig. 2B shows a reversing step.
Fig. 3 is a view showing another configuration of a porous body.
Fig. 4 is a view illustrating a process of a production method S20 of a tissue regeneration material: Fig. 4A shows a temporary step; and Fig. 4B shows a state when the whole porous body 11 is filled up with the cell suspension.
Fig. 5 is another view illustrating a process of the production method S20 of a tissue regeneration material: Fig. 5A shows a sandwiching step; and Fig. 5B shows a holding step.

### Modes for Carrying Out the Invention

The functions and benefits of the present invention described above will be apparent from the following modes for carrying out the invention. Hereinafter, the present invention will be described based on the embodiments shown in the drawings. However, the invention is not limited to these embodiments.

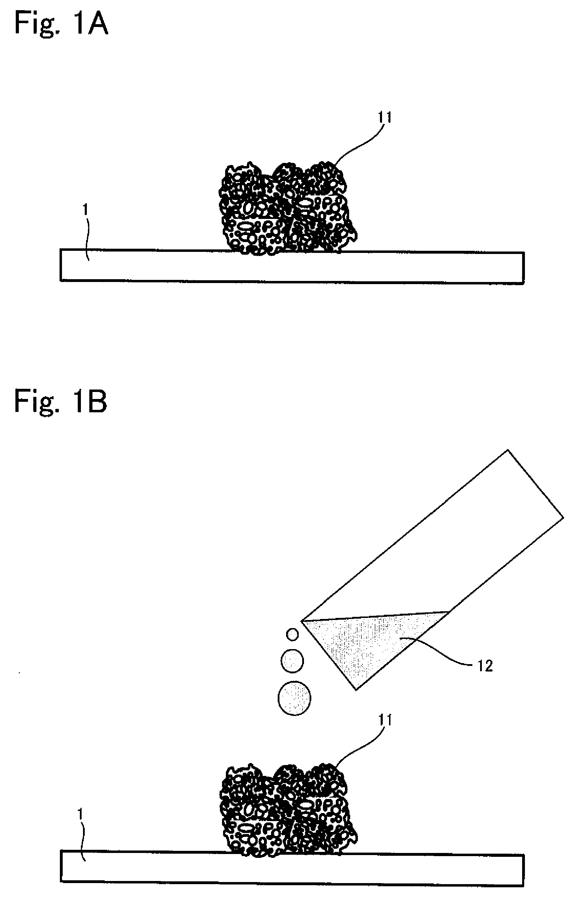

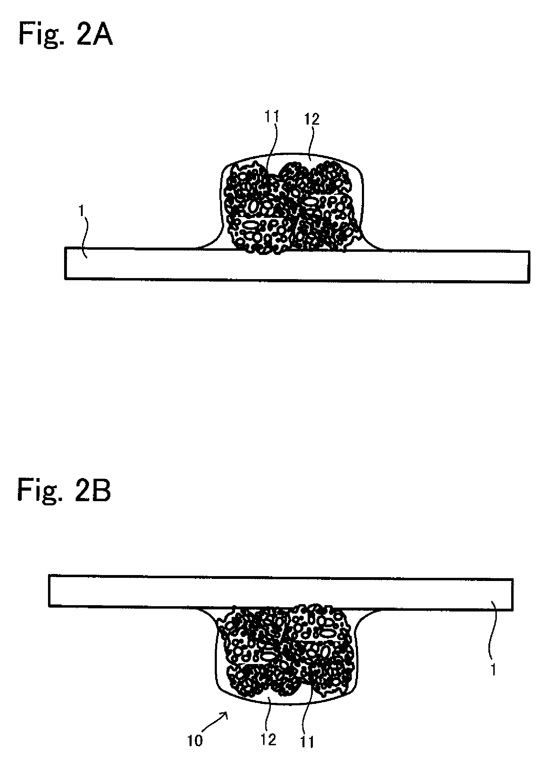

Figs. 1 and 2 are views illustrating the production method S10 of the tissue regeneration material (hereinafter referred to as a "production method S10") according to one embodiment. Figs. 1 and 2 show the scenes in the process of the production method S10. Herein, the tissue regeneration material 10 refers to a porous body 11 seeded with cells.

The production method S10 comprises a placement step S11, a cell suspension introduction step S12, and a reversing step S13.

The placement step S11 is a step of placing a porous body 11 on a holding plate 1, as shown in Fig. 1A.

Herein, the holding plate 1 is preferably a resin plate or a glass plate having a contact angle to water of 15 to 90°. Such a holding plate 1 can be obtained by processing into a plate shape a glass material used for a petri dish, flask, multiwell, etc., which have been conventionally used as a container for static culture of cells, or a resin material such as polystyrene, polyethylene, and polyethylene terephthalate. These materials sometimes have a high hydrophobic property. Therefore, it is preferable to modify the surface, as necessary, by a chemical treatment such as a corona treatment, γ ray treatment, argon ray treatment and plasma treatment etc., to introduce a polar group to the surface of the holding plate to be contacted with the porous body 11, increase its hydrophilicity, and make the surface have a contact angle to water of 15 to 90°. Further, a ceramic coating may be formed on the surface to be contacted. It is preferable for the surface to be contacted to be smooth; however, it may have grooves, streaky protrusions or the like having a height of several hundred micrometers.

The porous body 11 is formed of a bioabsorbable polymer material, and has the following characteristics at 23°C and 50% humidity: a thickness (a size in the top to bottom direction when placed on the holding plate 1) of 2 to 100 mm, preferably 2.1 to 70 mm; an interconnected porous structure with a pore diameter of 180 to 3500 µm and an average pore diameter of 350 to 2000 µm; a porosity of 60 to 95%; and a compressive strength of 0.05 to 1 MPa. It should be noted that the pore diameter of the porous body in the present invention does not take into account micropores of less than 10 µm that allow only liquid to penetrate therethrough, and means that 80% or more of the pores with a pore diameter of 10 µm or more in the whole porous body have a pore diameter of 180 to 3500 µm. Herein, the "porosity" is a value calculated from the weight of the porous body in comparison to the weight of a lump of raw material of the bioabsorbable polymer material used, the porous body having the same volume as that of the lump of raw material of the bioabsorbable polymer material used.

In this case, if the porosity is less than 60%, efficiency of cell growth and differentiation degrades. If it exceeds 95%, the strength of the porous body degrades. Therefore, the porosity is more preferably 80 to 90%. In addition, if the pore diameter is less than 180 µm, it is difficult to permeate the cells freely, preventing the cells to be seeded sufficiently into the porous body. On the other hand, if the pore diameter is more than 3500 µm, the strength of the porous body degrades.

Further, the average pore diameter is preferably 540 to 1200 µm.

As for the above compressive strength, if the compressive strength is less than 0.05 MPa, the porous body is caused to shrink by the surface tension generated from the cell suspension thereto or by the extension stress of cells. On the other hand, it is technically difficult to make a porous body having a compressive strength of over 1 MPa. The "compressive strength" herein refers to compressive fracture strength generated at a time of compressing a cylindrical test piece in a size of 10 mm diameter × 2 mm height at a cross head speed of 1 mm/min.

As for the thickness of the porous body, there is little need to use a porous body having a thickness of less than 2 mm since the purpose is to permeate the cell suspension sufficiently into the central part of the porous body. On the other hand, if the thickness exceeds 100 mm, it is difficult to introduce the cells or to culture the cells for a long period.

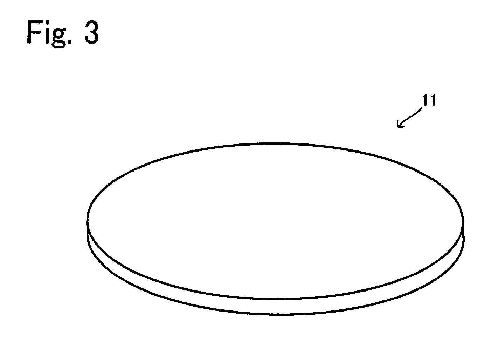

The shape of such a porous body is basically cubic as shown in Figs. 1 and 2, and in addition preferably semicircular or dome-shaped. However, it is not particularly limited as long as the cells can be seeded onto the porous body by arranging the above described holding plate 1 thereon in the gravity direction through the reversing step S13 and keeping the porous body still in the air in a state that the weight of the holding plate 1 is not applied to the porous body 11. It may be disc-shaped as shown in Fig. 3 for example.

At this time, the area of the base (the area of the surface which contacts the holding plate 1) of the porous body is preferably large enough in relation to the thickness thereof. In specific, the area of the base is preferably 0.5 to 200 cm² in terms of the range of the volume 1 to 50000 cm³ of the porous body. If the area of the base is less than 0.5 cm² or if it exceeds 200 cm², it is difficult to seed the cells with the porous body hung on the holding plate. The porous body may have powder-formed calcium phosphate, for example, hydroxyapatite or β-tricalcium phosphate dispersed in its bioabsorbable polymer material.

The material to constitute the porous body may be any without particular limitations as long as it is a bioabsorbable polymer material and can maintain its configuration inside the body for a certain period. Examples thereof include at least one selected from the followings, which have been conventionally used: polyglycolic acid; polylactic acid; a copolymer of lactic acid and glycolic acid; poly-ε-caprolactone; a copolymer of lactic acid and ε-caprolactone; polyamino acid; polyortho ester; and a copolymer thereof. Among these, polyglycolic acid, polylactic acid, and a copolymer of lactic acid and glycolic acid are most preferred as being approved by US Food and Drug Administration (FDA) as a polymer harmless to the human body and in view of their actual performance. The weight average molecular weight of the bioabsorbable polymer material is preferably 5000 to 2000000, and more preferably 10000 to 500000.

The production method of the porous body is not particularly limited. However, the production method may be for example as follows: mixing, in a substantially uniform manner, a particulate substance having a particle diameter of 100 to 2000 µm into a solution of a bioabsorbable polymer material dissolved in an organic solvent and freezing the mixture, the particulate substance not dissolving in this organic solvent but dissolving in a liquid that does not dissolve the bioabsorbable polymer material; thereafter drying the mixture to remove the organic solvent; thereby producing a porous bioabsorbable polymer which has a porous structure with a pore diameter of 5 to 50 µm and contains the particulate substance; pulverizing this porous bioabsorbable polymer with a mill or the like; then removing the particulate substance by dissolving it in the liquid that does not dissolve the bioabsorbable polymer; thereafter sifting it to make a bioabsorbable granular porous material having an average particle size of 100 to 3000 µm; and then putting the bioabsorbable granular porous material into a container having a predetermined shape to pressurize and heat it.

Descriptions of the production method S10 will be continued. Through the placement step S11, the porous body 11 is arranged on the surface of the holding plate 1. Next in the cell suspension introduction step S12, a cell suspension 12 is introduced into the porous body 11 for example by dripping or injection, as shown in Fig. 1B. Thereby, the cell suspension 12 permeates into the porous body 11 and fills up the whole porous body 11, as shown in Fig. 2A. At this time, if the amount of the cell suspension 12 is too large in relation to the volume of the porous body 11, it tends to be difficult to carry out the below described reversing step S13 or the culture efficiency tends to degrade. Therefore, it is preferable to introduce the cell suspension in an amount that can immerse the whole porous body 11 therein and that does not leak from the porous body 11 excessively.

The cell suspension to be introduced is arranged to have a cell concentration of 5 × 10⁶ to 1 × 10⁸ cell/ml. In addition, as to the cells to be cultured, various cells may be used without restrictions. Examples include epidermal cells, keratinocytes, fat cells, hepatocytes, nerve cells, neurogliacytes, astroglias, epitheliocytes, breast epidermal cells, chicken cells, endothelial cells, mesenchymal cells, dermal fibroblasts, mesothelial cells, osteoprogenitor cells, smooth muscle cells, striated muscle cells, ligament fibroblasts, tendon fibroblast, chondrocytes, bone-marrow cells, osteoblasts, tooth germ cells, periodontal ligament cells, dental pulp cells, somatic stem cells, and ES cells.

Next, through the reversing step S13, the holding plate 1 and the porous body 11 comprising the cell suspension 12 are reversed from the state shown in Fig. 2A, such that the holding plate 1 is on the upper side of the porous body 11, as shown in Fig. 2B. That is, when seen in the gravity direction, the holding plate 1 is on the upper side of the porous body 11, and they are kept still in the air in a state that the weight of the holding plate 1 is not applied to the porous body 11. With the holding plate 1 and the porous body 11 kept still in this state, the cells that have been introduced are adhered to the inner walls of the pores in the porous body 11, thereby completing seeding thereof. The obtained is the tissue regeneration material 10. The time to keep the porous body 11 still in the air in order to adhere the cells thereto varies depending on the material of the porous body and the kind of cell to be seeded; however, it is generally 20 to 300 minutes.

According to the production method S10 described above, the porous body 11 has relatively large pores and therefore the cell suspension can properly permeate into the porous body 11. And finally the cell suspension 12 can be kept in the porous body in a way not leaking therefrom, by arranging the holding plate 1 on the porous body 11 as described above when seen in the gravity direction and keeping them still in the air in the state that the weight of the holding plate 1 is not applied to the porous body 11. Accordingly, it is possible to introduce the cell suspension 12 stably without wasting it and seed the cells into the porous body. Thereafter, the tissue regeneration material, which is the porous material seeded with cells, can culture the cells by a conventional method.

In addition, in order to realize such a production method, it is preferable to use a porous body seeded with a cell suspension as described above. Thereby, a tissue regeneration material can be made which can produce the above advantageous effects.

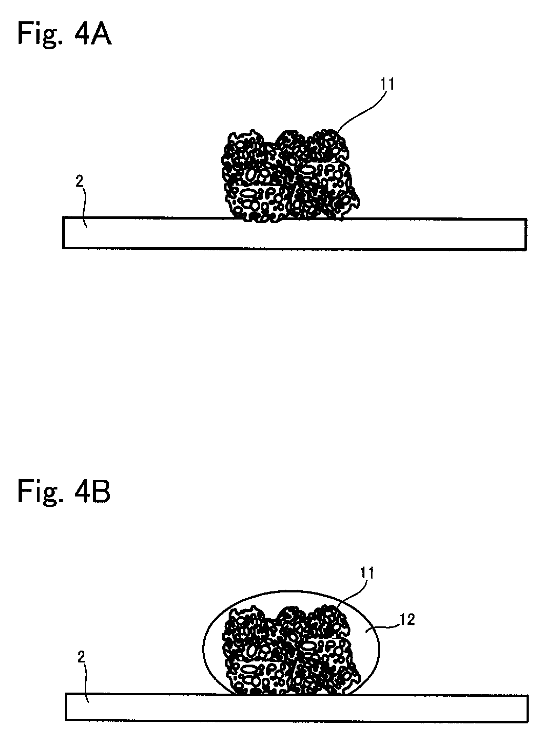

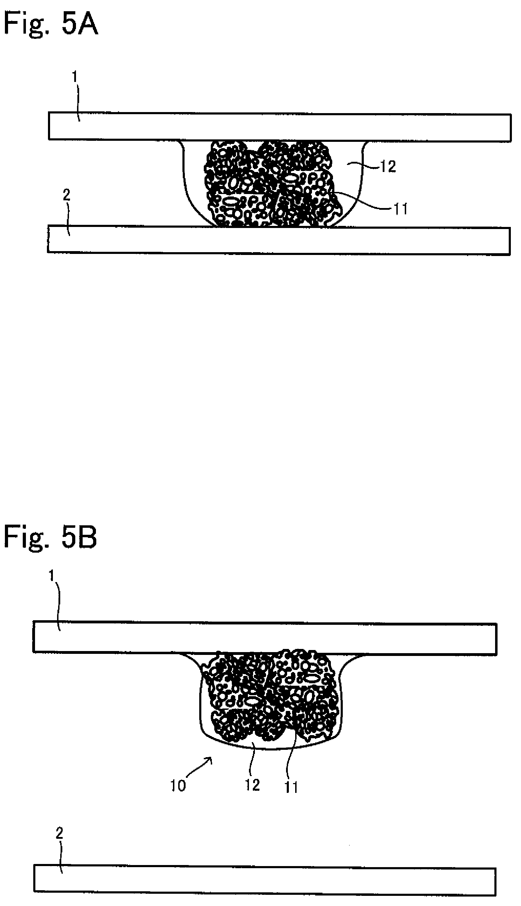

Figs. 4 and 5 are views illustrating a production method S20 of a tissue regeneration material (hereinafter sometimes referred to as a "production method S20") according to another embodiment. Figs. 4 and 5 show the scenes in the process of the production method S20. In the present embodiment, the same elements as those described in the above production method of one embodiment are given the same numerals, and descriptions thereof will be omitted.

The production method S20 comprises: a temporary placement step S21; a cell suspension introduction step S22; a sandwiching step S23; and a holding step S24.

The temporary placement step S21 is, as shown in Fig. 4A, a step of placing the porous body 11 onto a temporary placement plate 2, which is a plate member having high water repellency. The temporary placement plate 2 is preferably a resin plate or a glass plate having a contact angle to water of more than 90°.

In the cell suspension introduction step S22, the cell suspension 12 is introduced into the porous body 11, as in the above described cell suspension introduction step S12. Through this, the cell suspension 12 permeates into the porous body 11 to fill up the whole porous body 11, as shown in Fig. 4B.

The sandwiching step S23 is a step of contacting the porous body 11 filled up with the cell suspension to a surface of the holding plate 1 in a manner sandwiching the porous body 11 between the holding plate 1 and the temporary placement plate 2, as shown in Fig. 5A.

The holding step S24 is a step of pulling up the holding plate 1 contacted with the porous body 11, holding the porous body 11 on the holding plate 1 side, and releasing it from the temporary placement plate 2, as shown in Fig. 5B. Through this, as in one embodiment described above, the holding plate 1 is arranged to be on the porous body 11 when seen in the gravity direction, and they are kept still in the air in the state that the weight of the holding plate 1 is not applied to the porous body 11. With the porous body 11 kept still in this state, the cells that have been introduced are adhered to the inner wall of the porous body 11, thereby completing seeding thereof. The obtained is the tissue regeneration material 10.

In this case, the temporary placement plate 2 is constituted by a water-repellent plate; and the holding plate 1 is constituted by a hydrophilic plate. Therefore, the above described holding and releasing of the porous body filled with the cell suspension can be properly done.

### Examples

Hereinafter, the present invention will be described more in detail with Examples, but it is not limited to these examples.

### <Preparation of Implant Cells>

### Implant Cells A: Mesenchymal Stem Cells Harvested from Human Iliac Bone Marrow

Cells harvested from human iliac bone marrow were suspended in a DMEM culture medium supplemented with 10% FBS and thereafter seeded on a culture dish at nucleated cells of 1 × 10⁵ cell/10 cm diameter. The cells were cultured under the presence of 5% carbon dioxide gas at 37°C. The culture medium was changed on the third day to remove non-adherent cells (hematopoietic cells) . After that, the culture medium was changed every three days. From the fifth day, bFGF was added to the culture medium in an amount of 3 ng/ml. Around the 10th day, the cells were proliferated to be nearly confluent. These culture dishes were incubated for 5 minutes with trypsin (0.05%) and EDTA (0.2 mM) to isolate the cells. The number of cells was measured by Coulter Counter (ZI Single, manufactured by Coulter Corporation), and the cells were seeded at a density of 5000 cell/cm². This operation was repeated, and the third passage cells obtained from the second subculture dish nearly confluent were used.

### Implant Cells B: Mesenchymal Stem Cells Harvested from Human Jaw Bone Marrow

Cells harvested from human jaw bone marrow were suspended in a DMEM culture medium supplemented with 10% FBS and thereafter seeded on a culture dish at nucleated cells of 1 × 10⁵ cell/10 cm diameter. The cells were cultured under the presence of 5% carbon dioxide gas at 37°C. The culture medium was changed on the third day to remove non-adherent cells (hematopoietic cells) . After that, the culture medium was changed every three days. From the fifth day, bFGF was added to the culture medium in an amount of 3 ng/ml. Around the 10th day, the cells were proliferated to be nearly confluent. These culture dishes were incubated for 5 minutes with trypsin (0.05%) and EDTA (0.2 mM) to isolate the cells. The number of cells was measured by Coulter Counter (ZI Single, manufactured by Coulter Corporation), and the cells were seeded at a density of 5000 cell/cm². This operation was repeated, and the third passage cells obtained from the second subculture dish nearly confluent were used.

### Implant Cells C: Chondrocytes Harvested from Human Auricular Cartilage

A cell suspension was obtained by cutting a human auricular cartilage into small pieces with a surgical knife and treating them with collagenase at 37°C for 30 minutes. The cell suspension was suspended in a DMEM culture medium supplemented with 10% FBS and thereafter seeded on a culture dish at nucleated cells of 1 × 10⁵ cell/10 cm diameter. The cells were cultured under the presence of 5% carbon dioxide gas at 37°C. The culture medium was changed on the third day to remove non-adherent components (cells and tissue slices). After that, the culture medium was changed every three days. From the fifth day, bFGF was added to the culture medium in an amount of 3 ng/ml. Around the 10th day, the cells were proliferated to be nearly confluent. These culture dishes were incubated for 5 minutes with trypsin (0.05%) and EDTA (0.2 mM) to isolate the cells. The number of cells was measured by a Coulter Counter (ZI Single, manufactured by Coulter Corporation), and the cells were seeded at a density of 5000 cell/cm². This operation was repeated, and the third passage cells obtained from the second subculture dish nearly confluent were used.

### <Preparation of Porous Body>

### Porous Body A: Block of Polylactic Acid/Glycolic Acid Copolymer (PLGA)

A DL-lactic acid/glycolic acid copolymer having a molecular weight of 250000 was dissolved in dioxane, and thereafter the solution was mixed with sodium chloride having a particle size of around 500 µm. Then the mixture was freeze-dried, and was pulverized and desalinated to thereby obtain a powder-formed material. The powder-formed material thus obtained was molded by compression heating. Thereby, a block-shaped porous body was produced, which was made of a bioabsorbable synthetic polymer and had an average pore diameter of 540 µm, a porosity of 90%, a compressive strength of 0.2 MPa, a diameter of 9 mm, and a thickness of 3 mm.

Porous Body B: Block of Polylactic Acid/Glycolic Acid Copolymer (PLGA)

A DL-lactic acid/glycolic acid copolymer having a molecular weight of 250000 was dissolved in dioxane; thereafter hydroxyapatite powder having a particle size of 10 µm was dispersed in the solution; and the resultant was mixed with sodium chloride having a particle size of around 500 µm. Then the mixture was freeze-dried, and was pulverized and desalinated to thereby obtain a powder-formed material. The powder-formed material thus obtained was molded by compression heating. Thereby, a block-shaped porous body was produced, which was made of a bioabsorbable synthetic polymer and calcium phosphate and had an average pore diameter of 540 µm, a porosity of 90%, a compressive strength of 0.2 MPa, a diameter of 13 mm, and a thickness of 5 mm.

### <Example 1>

The porous body A was placed onto a glass plate (having a size of 100 mm × 100 mm × 2 mm and a contact angle to water of 28°), with the disc-shaped bottom surface of the porous body A contacted to the upper surface of the glass plate. The implant cells A, which were suspended in a chondrogenic differentiation medium (αMEM; glucose 4.5 mg/ml; 10⁻⁷ M dexamethasone; 50 µg/ml ascorbic acid-2-phosphoric acid; 10 ng/ml TGF-β; 6.25 µg/ml insulin; 6.25 µg/ml transferrin; 6.25 ng/ml selenic acid; 5.33 µg/ml linoleic acid; 1.25 mg/ml bovine serum albumin) at a concentration of 2 × 10⁷ cell/ml, were introduced into the porous body A in an amount of 0.19 ml by dripping. With the glass plate reversed to be upside down, the cells were seeded on the porous body A by adhering the cells to the material for 30 minutes under the conditions of 37°C, 100% humidity, and 5% CO₂, to thereby obtain a tissue regeneration material.

Thereafter, the tissue regeneration material was put into a centrifuge tube of 50 ml filled with a chondrogenic differentiation medium, and the cells were cultured at 37°C for four weeks while changing the culture medium every three days. Thereby, a cell implant for cartilage tissue regeneration was produced.

### <Example 2>

The porous body B was placed onto a plasma-treated polystyrene plate (having a size of 50 mm × 50 mm × 2 mm and a contact angle to water of 71°), with the disc-shaped bottom surface of the porous body B contacted to the upper surface of the plasma-treated polystyrene plate. The implant cells B, which were suspended in a osteogenic differentiation medium (αMEM; glucose 4.5 mg/ml; 10% FBS; 10⁻⁷ M dexamethasone; 50 µg/ml ascorbic acid-2-phosphoric acid; 10 mM β-glycerophosphoric acid)at a concentration of 5 × 10⁷ cell/ml, were introduced into the porous body B in an amount of 0.67 ml by dripping. With the plasma-treated polystyrene plate reversed to be upside down, the cells were seeded on the porous body B by adhering the cells to the material for 100 minutes under the conditions of 37°C, 100% humidity, and 5% CO₂, to thereby obtain a tissue regeneration material.

Thereafter, the tissue regeneration material was put into a centrifuge tube of 50 ml filled with an osteogenic differentiation medium, and the cells were cultured at 37°C for two weeks while changing the culture medium every three days. Thereby, a cell implant for bone tissue regeneration was produced.

### <Example 3>

The porous body B was placed onto a plasma-treated acrylic plate (having a size of 50 mm × 50 mm × 2 mm and a contact angle to water of 58°), with the disc-shaped bottom surface of the porous body B contacted to the upper surface of the plasma-treated acrylic plate. The implant cells C, which were suspended in a chondrogenic differentiation medium (αMEM; glucose 4.5 mg/ml; 10⁻⁷ M dexamethasone; 50 µg/ml ascorbic acid-2-phosphoric acid; 10 ng/ml TGF-β; 6.25 µg/ml insulin; 6.25 µg/ml transferrin; 6.25 ng/ml selenic acid; 5.33 µg/ml linoleic acid; 1.25 mg/ml bovine serum albumin) at a concentration of 2 × 10⁷ cell/ml, were introduced into the porous body B in an amount of 0.67 ml by dripping. With the plasma-treated acrylic plate reversed to be upside down, the cells were seeded on the porous body B by adhering the cells to the material for 30 minutes under the conditions of 37°C, 100% humidity, and 5% CO₂, to thereby obtain a tissue regeneration material.

Thereafter, the tissue regeneration material was put into a centrifuge tube of 50 ml filled with a chondrogenic differentiation medium, and the cells were cultured at 37°C for four weeks while changing the culture medium every three days. Thereby, a cell implant for cartilage tissue regeneration was produced.

### <Example 4>

The porous body A was placed onto a water-repellent glass plate (having a size of 50 mm × 50 mm × 2 mm and a contact angle to water of 114°). The implant cells B, which were suspended in a culture osteogenic differentiation medium (αMEM; glucose 4.5 mg/ml; 10% FBS; 10⁻⁷ M dexamethasone; 50 µg/ml ascorbic acid-2-phosphoric acid; 10 mM β-glycerophosphoric acid) at a concentration of 5 × 10⁷ cell/ml, were introduced into the porous body A in an amount of 0.19 ml by dripping. A plasma-treated polystyrene plate (having a size of 50 mm × 50 mm × 2 mm and a contact angle to water of 71°) was slowly put on the porous body A, in a direction from the upper side of the glass plate on which the porous body A was placed, so as to sandwich the porous body A together with the glass plate and to have the porous body contacted to the surface of the plasma-treated polystyrene plate. The porous body A was released from the glass plate by slowly pulling up the plasma-treated polystyrene plate contacted with the porous body, while it was kept on the plasma-treated polystyrene plate side. The porous body A had the plasma-treated polystyrene plate on the upper side thereof when seen in the gravity direction, and was kept still in the air in a state that the weight of the plasma-treated polystyrene plate was not applied thereto. With this reversed state, the cells were seeded on the porous body A by adhering the cells to the material for 100 minutes under the conditions of 37°C, 100% humidity, and 5% CO₂, to thereby obtain a tissue regeneration material.

Thereafter, the tissue regeneration material was put into a centrifuge tube of 50 ml filled with an osteogenic differentiation medium, and the cells were cultured at 37°C for two weeks while changing the culture medium every three days. Thereby, a cell implant for bone tissue regeneration was produced.

### Description of the Reference Numerals

- 1: holding plate
- 2: temporary placement plate
- 10: tissue regeneration material
- 11: porous body
- 12: cell suspension

## Claims

1. A production method for producing a tissue regeneration material comprising:
contacting a bottom surface of a porous body to a surface of a holding plate being a resin plate or a glass plate having a contact angle to water of 15 to 90°, the porous body being formed of a bioabsorbable polymer material selected from the group consisting of polyglycolic acid, polylactic acid and a copolymer of lactic acid and glycolic acid, poly-ε-caprolactone, a copolymer of lactic acid and ε-caprolactone, polyamino acid, polyortho ester, and a copolymer thereof, said porous body having a thickness of 2 to 100 mm, an area of the bottom surface of the porous body to be contacted with a holding plate of 0.5 to 200 cm², an interconnected porous structure with a pore diameter of 180 to 3500 µm and an average pore diameter of 350 to 2000 µm, a porosity of 60 to 95%, and a compressive strength of 0.05 to 1 MPa, and being filled with a cell suspension having a cell density of 5 × 10⁶ to 1 × 10⁸ cell/ml; and
the method further comprising reversing the holding plate and the porous body comprising the cell suspension such that the holding plate is on the upper side of the porous body when seen in the gravity direction, and keeping the porous body still in the air in a state that the weight of the holding plate is not applied to the porous body, to thereby seed cells on the porous body.

2. The production method according to claim 1, wherein the porosity of the porous body is 80 to 90%.

3. The production method according to claim 1 or claim 2, wherein the average pore diameter of the porous body is 540 to 1200 µm.

## Patentansprüche

1. Herstellungsverfahren zum Herstellen eines Geweberegenerationsmaterials, das Folgendes umfasst:
Inkontaktbringen einer unteren Oberfläche eines porösen Körpers mit einer Oberfläche einer Halteplatte, die eine Harzplatte oder Glasplatte ist, die einen Kontaktwinkel zu Wasser von 15 bis 90° aufweist, wobei der poröse Körper aus einem bioresorbierbaren Polymermaterial gebildet ist, das aus der Gruppe bestehend aus Polyglycolsäure, Polylactid und einem Copolymer aus Milchsäure und Glycolsäure, Poly-ε-Caprolactam, einem Copolymer aus Milchsäure und ε-Caprolactam, Polyaminosäure, Polyorthoester und einem Copolymer desselben ausgewählt ist, wobei der poröse Körper eine Dicke von 2 bis 100 mm, eine Fläche der mit einer Halteplatte in Kontakt zu bringenden unteren Oberfläche des porösen Körpers von 0,5 bis 200 cm², eine untereinander verbundene poröse Struktur mit einem Porendurchmesser von 180 bis 3500 µm und einem durchschnittlichen Porendurchmesser von 350 bis 2000 µm, eine Porosität von 60 bis 95 % und eine Druckfestigkeit von 0,05 bis 1 MPa aufweist und wobei der poröse Körper mit einer Zellsuspension mit einer Zelldichte von 5 x 10⁶ bis 1 x 10⁸ Zellen/ml gefüllt ist; und
wobei das Verfahren ferner ein Umkehren der Halteplatte und des porösen Körpers umfassend die Zellsuspension umfasst, sodass die Halteplatte in Schwerkraftrichtung betrachtet auf der Oberseite des porösen Körpers angeordnet ist, und wobei der poröse Körper in einem Zustand, in dem das Gewicht der Halteplatte nicht auf den porösen Körper wirkt, ruhig in der Luft gehalten wird, um dadurch Zellen auf dem porösen Körper auszusäen.

2. Herstellungsverfahren nach Anspruch 1, wobei die Porosität des porösen Körpers 80 bis 90 % beträgt.

3. Herstellungsverfahren nach Anspruch 1 oder Anspruch 2, wobei der durchschnittliche Porendurchmesser des porösen Körpers 540 bis 1200 µm beträgt.

## Revendications

1. Procédé de production d'un matériau de régénération de tissus consistant à :
mettre en contact une surface inférieure d'un corps poreux avec une surface d'une plaque de support étant une plaque en résine ou une plaque de verre présentant un angle de contact à l'eau de 15 à 90°, le corps poreux étant formé d'un matériau polymère bioabsorbable sélectionné parmi le groupe constitué d'acide polyglycolique, acide polylactique et un copolymère d'acide lactique et d'acide glycolique, poly-ε-caprolactone, un copolymère d'acide lactique et ε-caprolactone, polyamino acide, polyortho ester, et un copolymère de ceux-ci, ledit corps poreux ayant une épaisseur de 2 à 100 mm, une zone de la surface inférieure du corps poreux à mettre en contact avec un plaque de support de 0,5 à 200 cm², une structure poreuse interconnectée avec un diamètre de pore de 180 à 3500 µm et un diamètre de pore moyen de 350 à 2000 µm, une porosité de 60 à 95 %, et une résistance à la compression de 0,05 à 1 MPa, et étant rempli d'une suspension cellulaire ayant une densité de cellule de 5 x 10⁶ à 1 x 10⁸ cellule/ml ; et
le procédé consistant en outre à renverser la plaque de support et le corps poreux comprenant la suspension cellulaire de sorte que la plaque de support se trouve sur la face supérieure du corps poreux lorsque vu dans le sens de la gravité et à maintenir le corps poreux immobile dans l'air dans un état dans lequel le poids de la plaque de support n'est pas appliqué au corps poreux, afin d'ensemencer ainsi les cellules sur le corps poreux.

2. Procédé de production selon la revendication 1, dans lequel la porosité du corps poreux est de 80 à 90 %.

3. Procédé de production selon la revendication 1 ou 2, dans lequel le diamètre de pore moyen du corps poreux est de 540 to 1200 µm.
